# FASCICULE DE BREVET EUROPEEN

(11) **EP 3 294 234 B1**
(45) Date de publication et mention de la délivrance du brevet: **04.12.2019**
(21) Numéro de dépôt: 16703344.8
(22) Date de dépôt: 18.01.2016
(51) Int. Cl.: A61F 5/01, A61F 5/058, A61F 5/10, A61F 5/04

(54) **DISPOSITIF DE MAINTIEN ET/OU DE PROTECTION DE DOIGTS OU D'ORTEILS**
VORRICHTUNG ZUR UNTERSTÜTZUNG UND/ODER ZUM SCHUTZ VON FINGERN ODER ZEHEN
DEVICE FOR SUPPORT AND/OR PROTECTION OF FINGERS OR TOES

(30) Priorité: 27.01.2015 FR 1550629; 08.04.2015 FR 1553015
(43) Date de publication de la demande: 21.03.2018
(73) Titulaire: Creation XXI S.A.S, 64500 Ciboure (FR)
(72) Inventeur: SIVY Yann, 64530 Ger (FR); BLOEDT Laurent, Le Bouscat 33110 (FR)
(74) Mandataire: Vienne, Aymeric Charles Emile
(86) Numéro de dépôt international: PCT/FR2016/050088
(87) Numéro de publication internationale: WO 2016/120543

(56) Documents cités:
- CN-U- 202 497 582
- DE-U1-202006 011 664
- US-A- 5 267 945
- US-A1- 2007 017 004
- US-A1- 2011 046 531
- US-A1- 2013 326 790

## Description

L'invention concerne le domaine des appareils de maintien des doigts et des orteils tels que les attelles ou les bandes élastiques permettant d'immobiliser plusieurs doigts ou orteils suite à une blessure notamment articulaire nécessitant un repos des articulations correspondantes.

Plus particulièrement, l'invention concerne un dispositif de maintien et/ou de protection des doigts ou des orteils permettant une immobilisation totale ou partielle desdits doigts ou orteils les uns par rapport aux autres.

### [Art antérieur]

Les dispositifs de maintien ou de contention des doigts ou des orteils permettant d'immobiliser lesdits doigts ou orteils suite à une fracture ou tout type de blessure notamment de type articulaire sont bien connus. Il est courant d'utiliser des bandes élastiques ou straps venant s'appliquer autour des doigts blessés, entourant lesdits doigts de manière à former un ensemble sensiblement cylindrique généralement maintenu en place par des attaches adhésives ou des scratchs.

Ces bandes élastiques présentent l'inconvénient de se salir et de s'user très rapidement, et nécessitent donc d'être changées régulièrement, notamment du fait de la transpiration, de l'eau en cas de pluie ou du fait de l'hygiène corporelle, et de leur fragilité. Par ailleurs, la mise en place ainsi que le retrait des bandes élastiques est souvent contraignant et douloureux pour l'utilisateur. Généralement réalisée par l'utilisateur lui-même lors de blessures de faible gravité, la mise en place des bandes élastiques est laborieuse et effectuée avec une seule main, c'est-à-dire la main non blessée. La bande n'est donc pas enroulée de manière régulière, ce qui fragilise l'ensemble, le rend moins durable et moins efficace. Le retrait des bandes élastiques est douloureux, d'une part à cause des attaches adhésives qu'il faut décoller de la peau, et d'autre part du fait des multiples déplacements des doigts de la main blessée au fur et à mesure du déroulement des bandes, pouvant rallonger considérablement le temps de guérison de la blessure.

Afin de remédier à certains de ces inconvénients, le document US 2004/0144389 décrit un dispositif médical pour effectuer une syndactylie de doigts ou d'orteils, comprenant deux anneaux élastiques reliés par une barre de liaison souple mais pas élastique de sorte que, après la mise en place dudit dispositif médical sur les doigt ou orteils lésés, lesdits doigts ou orteils lésés peuvent glisser les uns par rapport aux autres. Cependant, ce dispositif médical ne comprend pas de système permettant de régler la taille et en particulier le diamètre des anneaux. Le caractère élastique des anneaux permet tout au plus d'assurer un maintien modéré des doigts ou orteils. Ainsi, il est impossible pour l'utilisateur de régler précisément ce dispositif lors de son utilisation afin qu'il corresponde à la morphologie de ses mains ou de ses pieds. Or la morphologie des mains et des pieds de l'utilisateur sont susceptibles de varier, non seulement en fonction de son âge, mais également en fonction du type de blessure pouvant faire varier le volume de ses mains ou de ses pieds au niveau des zones lésées.

Le document FR 2 578 740 décrit une orthèse dynamique de syndactylisation comprenant une pièce rigide d'immobilisation comportant deux ou trois alvéoles et une pièce souple autocollante de cerclage de la partie ouverte de l'orthèse. L'orthèse décrite dans ce document présente l'inconvénient de ne se positionner que sur les phalanges des doigts, permettant le fonctionnement des articulations, ce qui est généralement à proscrire dans le traitement des fractures ou autres types de traitements orthopédiques. En effet, bien que le temps de guérison puisse être raccourci, il ne s'agit généralement que de quelques jours sur des périodes de l'ordre de plusieurs semaines voire plusieurs mois. De plus, le risque d'une mauvaise guérison voire d'une aggravation de la blessure est bien présent, induisant le cas échéant un allongement considérable du temps de guérison. Par ailleurs, la bande souple autocollante assurant le cerclage de la partie ouverte de l'orthèse permet certes de faire varier le diamètre des alvéolages en fonction des propriétés de déformation du matériau souple utilisé pour sa fabrication, mais cette variation reste faible du fait que la pièce rigide est peu ou pas déformable, et que la bande souple autocollante est une pièce distincte de la pièce rigide. De plus, le caractère autocollant de la bande souple rend la liaison sensible notamment à l'usure et à l'humidité, comme décrit précédemment.

US 5 267 945 divulgue une attelle de doigt pour le traitement des blessures articulaires. US 2011/046531 divulgue un dispositif de correction d'extraversion d'orteil.

Des aspects de l'invention sont repris dans la revendication indépendante et des caractéristiques optionnelles sont reprises dans les revendications dépendantes.

L'invention a donc pour but de remédier aux inconvénients de l'art antérieur en proposant un dispositif de maintien et/ou de protection de doigts ou d'orteils permettant d'immobiliser totalement ou partiellement au moins deux doigts ou orteils. Le dispositif permet également de régler les dimensions des éléments enserrant lesdits doigts ou orteils afin de les faire correspondre à la morphologie de l'utilisateur. Le dispositif est en outre parfaitement maintenu en position sur la main ou le pied de l'utilisateur grâce à un système de fermeture empêchant tout mouvement dudit dispositif après sa mise en place, et peut être mis en place et retiré en toute simplicité. Le dispositif proposé n'est que très peu sensible à la salissure, l'usure et l'humidité, et ne nécessite donc pas d'être régulièrement changé.

### [Brève description de l'invention]

A cet effet, l'invention a pour objet un dispositif de maintien et/ou de protection d'au moins deux doigts ou orteils permettant d'immobiliser totalement ou partiellement lesdits doigts ou orteils les uns par rapport aux autres, le dispositif comprenant une bande se présentant sous la forme d'une boucle délimitant un espace de logement destiné à accueillir lesdits doigts ou orteils, ladite bande se terminant par deux extrémités formant deux parties d'un système de fermeture de la boucle, les deux parties comportant des pièces d'attaches complémentaires coopérant pour assurer un réglage et la fermeture la boucle, le dispositif étant principalement caractérisé en ce que la bande est en matériau polymère rigide et est munie à l'une de ses extrémités d'ergots et à l'autre extrémité d'orifices constituant les pièces d'attache du système de fermeture.

Selon d'autres caractéristiques optionnelles du dispositif :
- Les ergots sont de type boutons ou crochets sur une première partie du système de fermeture et en ce que les pièces complémentaires sont disposées de sorte que le système de fermeture permet le réglage de la longueur de la boucle par superposition de la première partie et de la deuxième partie et insertion des ergots dans les orifices ;
- La première partie du système de fermeture est munie d'une ou plusieurs rangées d'ergots, de préférence lesdites rangées d'ergots définissent un axe parallèle à l'axe longitudinal (A) de la bande ;
- La deuxième partie du système de fermeture est munie d'une ou plusieurs rangées d'orifices, de préférence lesdites rangées d'orifices définissent un axe parallèle à l'axe longitudinal (A) de la bande ;
- Le nombre d'ergots de la première partie du système de fermeture est inférieur ou égal au nombre d'orifices de la deuxième partie du système de fermeture ;
- Les ergots sont avantageusement des boutons en forme de champignons ou des crochets et les orifices ont un diamètre au moins égal au diamètre de la tête des champignons ou à la largeur des crochets ;
- La bande comprend en outre une première partie intermédiaire et une deuxième partie intermédiaire, lesdites parties intermédiaires étant aptes à se courber de manière à constituer les extrémités latérales de la boucle ;
- La bande comprend en outre une partie centrale délimitée par la première partie intermédiaire et la deuxième partie intermédiaire ;
- La bande comprend au moins un élément de séparation des doigts prévu au niveau de la partie centrale afin d'améliorer le confort des utilisateurs;
- L'élément de séparation des doigts comprend deux faces latérales et deux faces longitudinales, lesdites faces latérales étant évasées au niveau de leur partie inférieure de manière à former une concavité au niveau de la partie inférieure des deux faces longitudinales ;
- L'élément de séparation des doigts comprend un retrait réalisé dans la partie inférieure des faces latérales et traversant ledit élément de séparation sur toute sa longueur ;
- Avantageusement, la bande comprend une pluralité d'orifices de faible diamètre comparativement aux orifices du système de fermeture, permettant une régulation de la température et de l'humidité au niveau des doigts ou des orteils entourés par ladite bande ;
- La boucle est de forme sensiblement oblongue et de hauteur variable de sorte que la hauteur h de la première extrémité latérale de la boucle est inférieure à la hauteur H de la deuxième extrémité latérale de la boucle ;
- La bande est obtenue par moulage en une seule pièce, à partir d'une résine polymère ;
- La bande est obtenue par impression en trois dimensions en une seule pièce, à partir d'une résine polymère ;
- Les ergots et les orifices de la bande sont obtenus par l'étape de moulage ou d'impression en trois dimensions de la bande ;
- La bande et le système de fermeture constituent un seul et même élément en matériau plastique réalisé par moulage ou impression en trois dimensions.

D'autres avantages et caractéristiques de l'invention apparaitront à la lecture de la description suivante donnée à titre d'exemple illustratif et non limitatif, en référence aux Figures annexées qui représentent :
- La Figure 1, un schéma en vue de côté du dispositif selon l'invention
- La Figure 2, un schéma en perspective du dispositif selon l'invention, les deux parties du système de fermeture étant juxtaposées, le système de fermeture verrouillé, et les ergots étant constitués de boutons.
- La Figure 3, un schéma en vue de dessus du dispositif selon l'invention, la bande étant dépliée.
- La Figure 4, un schéma d'un mode de réalisation de juxtaposition des parties du système de fermeture « en décalé ».
- La Figure 5, un schéma en perspective du dispositif selon l'invention, les deux parties du système de fermeture étant juxtaposées, le système de fermeture verrouillé, et les ergots étant constitués de crochets.
- La Figure 6, un schéma en perspective du dispositif selon l'invention, muni d'un élément de séparation des doigts.
- La Figure 7, un schéma en perspective du dispositif selon l'invention, muni d'un élément de séparation des doigts selon une variante de réalisation.

### [Description détaillée de l'invention]

L'invention concerne un dispositif 1 de maintien et/ou de protection d'au moins deux doigts ou orteils permettant d'immobiliser totalement ou partiellement au moins deux doigts d'une main ou deux orteils d'un pied les uns par rapport aux autres. Les doigts ou orteils munis du dispositif selon l'invention restent cependant mobiles par rapport aux autres doigts ou orteils. Un tel dispositif est notamment utilisé dans le cadre d'un traitement orthopédique, qu'il s'agisse d'une immobilisation de longue durée ou pour une reprise sportive par exemple. Le dispositif peut être mis en place autour des phalanges et/ou des articulation des doigts ou orteils blessés, autorisant ou interdisant selon le cas le mouvement des articulations desdits doigts ou orteils blessés.

Dans la suite de la description, on parlera seulement des doigts et non des orteils afin de simplifier la lecture. Il doit cependant être compris que l'invention s'applique indifféremment aux doigts et aux orteils.

On désigne par « doigts blessés » les doigts ou orteils munis du dispositif selon l'invention. La blessure peut être de type articulaire ou non. Il peut s'agir notamment d'une fracture ou d'une entorse. Il peut également s'agir d'une plaie causée par une éraflure, une coupure, ou une piqûre par exemple, ou encore une brûlure, un hématome, ou une blessure suturée.

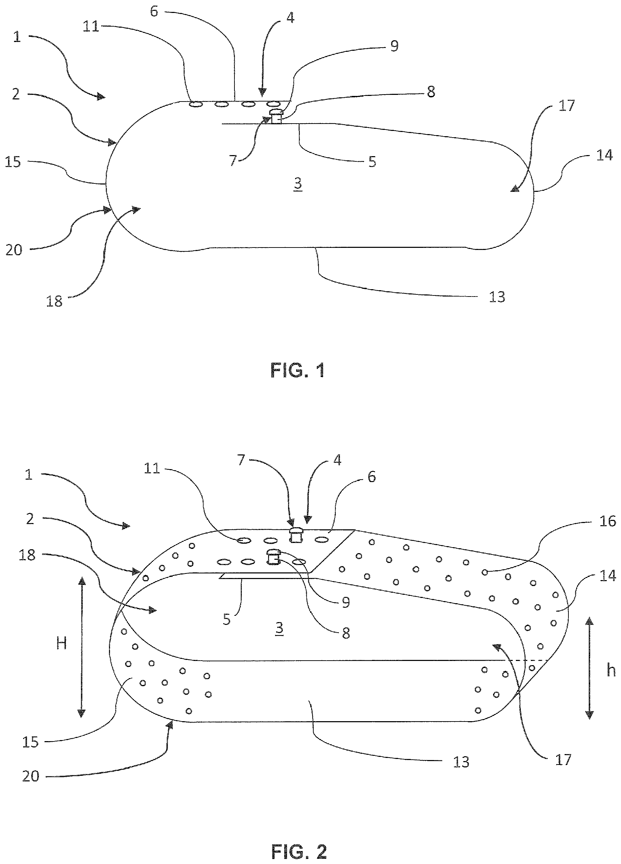

Le dispositif 1 de maintien et/ou de protection selon l'invention comprend une bande 2 apte à former une boucle 20. La boucle est de forme sensiblement oblongue, définit une première extrémité latérale 17 et une deuxième extrémité latérale 18, et délimite un espace de logement 3 des doigts, de sorte qu'une fois insérés dans ledit espace de logement, les doigts sont entourés par la bande formant ladite boucle. Selon un mode de réalisation préféré du dispositif représenté sur les Figures 1, 2 et 5, la hauteur de la boucle 20 est variable. Plus précisément, la hauteur de la boucle varie entre une valeur minimale h au niveau de la première extrémité latérale 17 et une valeur maximale H au niveau de la deuxième extrémité latérale 18, avec h inférieur ou égal à H.

Cette variation de hauteur permet de mieux épouser la forme des doigts lorsque lesdits doigts sont insérés dans l'espace de logement 3. En effet, le diamètre du majeur par exemple est généralement plus important que celui des trois autres doigts, si l'on exclut le pouce. L'annulaire a un diamètre sensiblement égal à celui du majeur. L'index a un diamètre plus faible que le majeur, et l'auriculaire a un diamètre plus faible que l'annulaire. Ainsi, lors de la mise en place du dispositif autour du majeur, de l'annulaire, et de l'auriculaire par exemple, la variation de hauteur de la boucle 20 correspond à la diminution de diamètre entre le majeur, l'annulaire, et l'auriculaire.

Bien entendu, la boucle 20 peut également avoir une forme parfaitement oblongue ou circulaire, notamment lorsque la bande 2 est destinée à n'entourer que le majeur et l'annulaire.

La bande 2 comprend un système de fermeture 4 de la boucle 20 comprenant une première partie 5 et une deuxième partie 6 correspondant aux extrémités de la bande. Les deux parties 5 et 6 comportent des pièces d'attaches complémentaires 7 et 11 qui coopèrent pour réaliser ladite fermeture de la boucle. Les pièces d'attaches complémentaires des deux parties 5 et 6 coopèrent également pour permettre un réglage de la longueur de la boucle 20 après mise en place autour des doigts.

Comme représentées sur les Figures 1 à 5, les deux parties du système de fermeture 4 constituent les deux extrémités de la bande 2. Les deux parties 5 et 6 du système de fermeture 4 font intégralement partie de la bande 2, de sorte que lesdites parties 5 et 6 du système de fermeture 4 et ladite bande 2 ne forment qu'une seule pièce.

La première partie 5 comporte un ou plusieurs ergots 7 destinés à venir se loger dans les ouvertures 11 de la deuxième partie 6 pour fermer la boucle 20. Suite à une blessure, le dispositif 1 selon l'invention est apposé sur la main ou le pied blessé de manière à ce que la bande 2 entoure les doigts ou orteils blessés. Les deux parties 5 et 6 du système de fermeture 4 sont disposées à l'aplomb l'une de l'autre puis les ergots 7 de la première partie 5 viennent se loger dans les ouvertures 11 de la deuxième partie 6 de manière à ce que les deux parties du système de fermeture soient superposées. La boucle 20 est ainsi fermée.

De préférence, la première partie 5 du système de fermeture 4 comporte une ou plusieurs rangées 10 d'un ou plusieurs ergots 7 disposées selon un axe parallèle à l'axe longitudinal (A) de la bande 2. Le nombre d'ergots peut être identique ou différent d'une rangée à une autre. De manière davantage préférée, la première partie 5 comporte deux ergots 7 symétriques par rapport à l'axe longitudinal (A) de la bande.

La deuxième partie 6 du système de fermeture 4 comporte une ou plusieurs ouvertures 11 destinées à accueillir les ergots 7 de la première partie 5 lors de la mise en place de la bande 2 autour des doigts blessés. De préférence, la deuxième partie 6 comporte une ou plusieurs rangées 12 d'une ou plusieurs ouvertures 11 disposées selon un axe parallèle à l'axe (A) de la bande 2. Le nombre d'ouvertures peut être identique ou différent d'une rangée à une autre. De manière davantage préférée, la deuxième partie 6 comporte deux rangées 12 de trois ouvertures, lesdites rangées étant symétriques par rapport à l'axe (A). Sur les Figures 2 à 5 sont ainsi représentés deux rangées 10.1 et 10.2 comprenant chacune un ergot 7, et deux rangées 12.1 et 12.2 comprenant chacune quatre ouvertures 11. L'axe longitudinal (A) est de préférence un axe de symétrie de la bande.

De préférence, les rangées 10 d'ergots 7 de la première partie 5 et les rangées 12 d'ouvertures 11 de la deuxième partie 6 sont alignées deux à deux selon un axe parallèle à l'axe (A), de sorte que, lors de l'insertion des ergots dans les ouvertures, les première et deuxième parties sont parfaitement juxtaposées.

Les ergots 7 de la première partie 5 et les ouvertures 11 de la deuxième partie 6 du système de fermeture 4 permettent de fermer la boucle 20 formée par la bande 2, et de régler la longueur de la boucle afin d'ajuster de manière optimale le dispositif selon l'invention aux doigts blessés de l'utilisateur. Le réglage de la longueur de la boucle permet de serrer ou de desserrer la bande 2 entourant les doigts blessés.

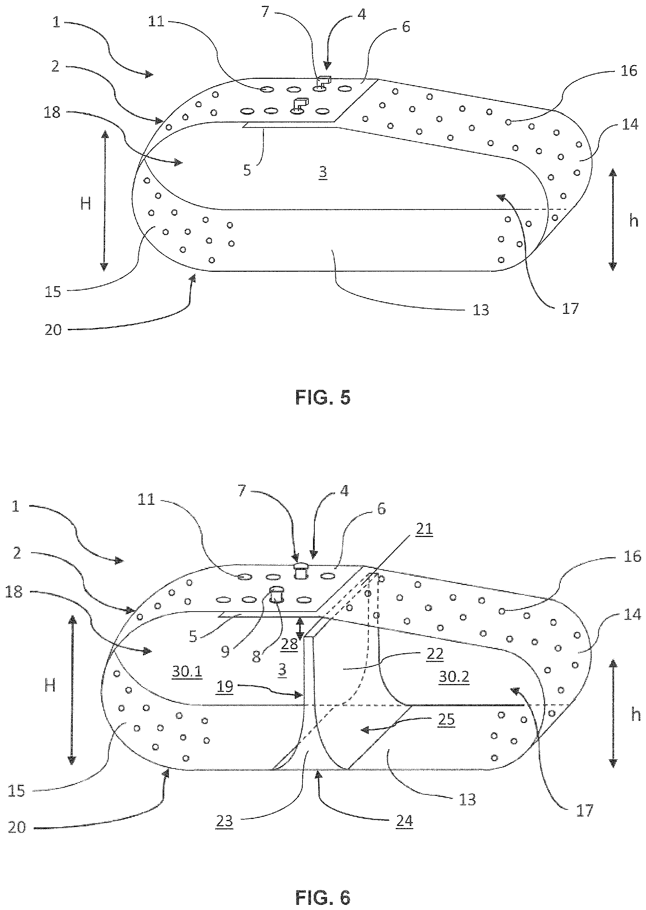

Les ergots 7 sont des excroissances réalisées sur la bande 2 pendant l'étape de moulage. Avantageusement, les ergots 7 sont des boutons en forme de champignons ou des crochets et les ouvertures 11 sont des orifices de diamètre au moins égal au diamètre de la tête des champignons ou à la largeur des crochets, réalisés pendant l'étape de moulage. Sur les Figures 1 à 4, les ergots sont des boutons en forme de champignons. Sur la Figure 5, les ergots sont des crochets.

Les ouvertures 11 sont réalisées sur la bande 2 pendant l'étape de moulage. Avantageusement, les ouvertures 11 sont des orifices ou des fentes de dimensions adaptées pour accueillir les ergots 7.

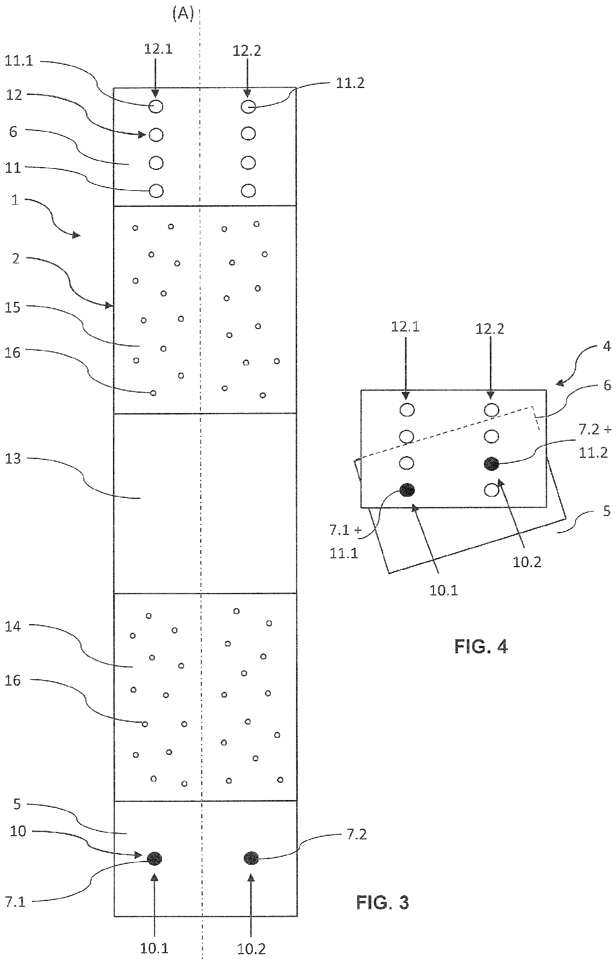

Lors de la mise en place du dispositif autour des doigts blessés, il est également possible de positionner les deux parties 5 et 6 du système de fermeture 4 « en décalé » l'une par rapport à l'autre, en référence à la Figure 4. La juxtaposition des deux parties 5 et 6 n'étant dès lors plus parfaite. Par exemple, on peut d'une part insérer l'ergot 7.1 de la première rangée d'ergots 10.1 dans la première ouverture 11.1 de la première rangée d'ouvertures 12.1, et d'autre part insérer l'ergot 7.2 de la deuxième rangée d'ergots 10.2 dans la deuxième ouverture 11.2 de la deuxième rangée d'ouvertures 12.2. Le dispositif 1 prend alors une forme évasée. Ce mode de réalisation peut permettre d'améliorer la tenue du dispositif sur les doigts blessés en s'adaptant au mieux à la forme des doigts blessés de l'utilisateur et à l'emplacement de la blessure sur lesdits doigts. Bien entendu ce mode de fermeture en décalé n'est pas limité à celui précédemment décrit, et toute variante de ce mode de fermeture est également envisageable, et de ce fait objet de la présente invention.

De plus, la première partie 5 du système de fermeture 4 peut comprendre un nombre d'ergots 7 égal, inférieur, ou supérieur au nombre d'ouvertures 11.

De préférence, la première partie 5 du système de fermeture 4 comprend un nombre d'ergots 7 inférieur au nombre d'ouvertures 11. Des bandes de tailles différentes peuvent être réalisées pour correspondre à la morphologie d'un adulte ou d'un enfant par exemple. Il peut être également prévu que le nombre d'ergots et d'ouvertures ne soient pas le même pour toutes les bandes, de manière à ce que le réglage soit toujours adapté et possible quel que soit l'utilisateur. Un grand nombre d'ouvertures permet en effet un réglage plus précis de la longueur de la boucle 20 et ainsi un meilleur ajustement du dispositif aux doigts blessés de l'utilisateur. De manière avantageuse, il est possible, une fois le dispositif mis en place autour des doigts blessés et les ergots 7 insérés dans les ouvertures 11, de découper le surplus de bande 2 dépassant au niveau des parties 5, 6 du système de fermeture 4.

La bande 2 comprend en outre une première partie intermédiaire 14 et une deuxième partie intermédiaire 15. Les deux parties intermédiaires sont chacune situées entre l'une des deux parties 5 ou 6 du système de fermeture 4, et une partie centrale 13. Les deux parties intermédiaires sont aptes à se courber afin que la bande 2 entoure les doigts de l'utilisateur, et constituent les extrémités latérales 17 et 18 de la boucle 20.

De plus, l'ouverture et la fermeture de la boucle 20, ainsi que le serrage et le desserrage de la bande 2, sont réalisés de manière simple et rapide, notamment par pression sur les extrémités latérales 17 et 18 de manière à les rapprocher l'une de l'autre. Lorsque plus aucune pression n'est appliquée sur les extrémités latérales, celles-ci reviennent en position d'équilibre par effet ressort. La structure de la bande 2 ainsi que le matériau utilisé pour la fabrication de ladite bande contribuent à cet effet ressort.

La bande 2 comprend en outre une partie centrale 13. La partie centrale est délimitée par les deux parties intermédiaires 14 et 15. Ladite partie centrale contribue à créer un effet ressort induisant une répulsion des deux parties intermédiaires 14 et 15 l'une par rapport à l'autre, et ainsi une répulsion des parties 5 et 6 du système de fermeture 4 l'une par rapport à l'autre. De cette manière, lors de la mise en place du dispositif autour des doigts blessés de l'utilisateur, le système de fermeture 4 est verrouillé grâce :
- Aux ergots 7 venant en butée contre la paroi des ouvertures 11, lesdits ergots étant avantageusement coiffés d'une tête 9 de plus grand diamètre que leur corps 8 ou constitués de crochets, afin d'éviter que le système de fermeture 4 ne se déverrouille par glissement des ergots 7 relativement aux ouvertures 11, et
- A l'effet ressort provoquant un retour des parties intermédiaires 14 et 15, et renforçant l'effet de butée des ergots 7 contre la paroi des ouvertures 11.

La bande 2 peut comprendre en outre un ou plusieurs éléments de séparation 19 des doigts, permettant de diviser l'espace de logement 3 en plusieurs espaces secondaires 30.1, 30.2. L'élément de séparation est situé au niveau de la partie centrale 13 de la bande. Ledit élément de séparation est lié ou fixé à la bande 2 par tout moyen de liaison ou de fixation connu, par exemple par collage ou avec des crochets, ou fait partie intégrante de ladite bande, en ce qu'il est moulé avec ladite bande en tant qu'élément constitutif de celle-ci.

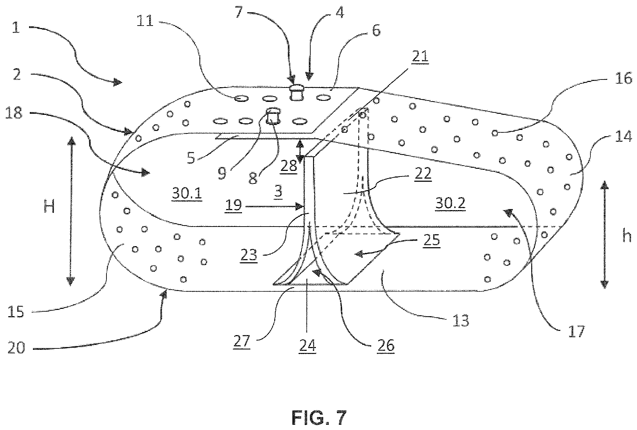

De manière avantageuse, et tel que représenté sur les Figures 6 et 7, la bande 2 comprend un unique élément de séparation 19 permettant de diviser l'espace de logement 3 en deux espaces secondaires 30.1, 30.2. Les deux espaces secondaires sont ainsi chacun délimités par la partie centrale 13, et l'une des première et deuxième parties intermédiaires 14,15, et sont partiellement séparés l'un de l'autre par l'élément de séparation 19, autorisant les deux espaces secondaires à communiquer l'un avec l'autre par un intervalle 28.

L'élément de séparation comprend une base 24 au contact de la partie centrale 13 de la bande 2 ou faisant partie intégrante de ladite partie centrale, ainsi qu'une face supérieure 21 au contact ou non avec l'une quelconque des deux parties 5,6 du système de fermeture 4 ou avec l'une quelconque des deux parties intermédiaires 14,15 de la bande 2. De préférence, la longueur de la base 24 correspond à la largeur de la partie centrale 13, bien qu'il soit également possible de prévoir que la base ait une longueur inférieure à la largeur de la partie centrale comme représenté sur la Figure 7.

L'élément de séparation 19 comprend en outre deux faces longitudinales 22 séparant totalement ou partiellement les espaces secondaires 30.1, 30.2 l'un de l'autre, ainsi que deux faces latérales 23. Les deux faces latérales sont avantageusement évasées au niveau de leur partie inférieure de manière à former une concavité 25. De préférence, la courbure de la concavité est prévue de manière à correspondre à celle des doigts insérés dans les espaces secondaires 30.1, 30.2 afin que ladite concavité épouse la forme des doigts de l'utilisateur, pour un confort optimal. Dans ce but, la courbure de la concavité 25 des faces longitudinales 22 peut être différente d'un élément de séparation à un autre. De la même manière, la courbure de la concavité des deux faces longitudinales d'un même élément de séparation peut être différente.

La face supérieure 21 de l'élément de séparation 19 peut être libre, en ce qu'elle n'est au contact d'aucun élément, de sorte que les espaces secondaires 30.1, 30.2 ainsi formés communiquent entre eux par l'intervalle 28.

Alternativement, l'élément de séparation 19 peut relier la partie centrale 13 de la bande 2 à l'une quelconque des deux parties 5,6 du système de fermeture 4, ou à l'une quelconque des deux parties intermédiaires 14,15 de la bande 2. Dans les deux cas précédents, les espaces secondaires 30.1, 30.2 ainsi formés ne communiquent pas entre eux et sont séparés l'un de l'autre par l'élément de séparation 19.

Selon une variante de réalisation du dispositif représentée sur la Figure 7, l'élément de séparation 19 comprend un retrait 26 réalisé au niveau de la partie inférieure des faces latérales 23 et traversant l'élément de séparation sur toute sa longueur. La base 24 est alors évidée. Ce mode de réalisation permet de rendre l'élément de séparation 19 plus flexible, facilitant la mise en place du dispositif sur les doigts de l'utilisateur et rendant ledit dispositif plus confortable pour l'utilisateur. Du fait de l'évidement de la base de l'élément de séparation, la longueur de ladite base est inférieure à la largeur de la bande 2, formant ainsi deux couloirs 27 parallèles entre eux.

La bande 2 comprend en outre des orifices 16 de faible diamètre comparativement aux ouvertures 11 de la deuxième partie 6 du système de fermeture 4. Les orifices 16 sont situés sur la partie centrale 13 et/ou sur les parties intermédiaires 14 et 15 et/ou sur les parties 5 et 6 du système de fermeture 4 et/ou sur l'élément de séparation 19. De préférence, les orifices secondaires 16 sont situés uniquement sur les parties intermédiaires 14 et 15 de la bande, tels que représentés sur les Figures 2, 3 et 5.

Les orifices 16 permettent une aération des doigts blessés. Ils permettent ainsi de réguler la température et d'améliorer l'hygiène desdits doigts blessés en évitant la transpiration. La présence d'orifices secondaires est particulièrement intéressante dans le cadre de blessures de type plaie. Le dispositif 1 est positionné autour des doigts blessés, par-dessus et en complément d'un pansement préalablement appliqué sur la plaie. Le dispositif contribue ainsi au maintien du pansement sur la plaie, et permet de protéger ladite plaie des chocs ou facteurs externes.

La bande 2 est obtenue par moulage à l'aide d'une résine polymère. La bande 2 est moulée en une seule pièce. De préférence, le moule comprend des zones de hauteurs différentes, de manière à obtenir une bande dont l'épaisseur des premières et deuxièmes parties 5 et 6 du système de fermeture 4 et de la partie centrale 13 est supérieure à l'épaisseur des parties intermédiaires 14 et 15. Lorsque la bande est obtenue par moulage, les ergots 7 sont de préférence des crochets.

Selon une variante de réalisation, la bande 2 peut être obtenue par impression en trois dimensions, dite impression 3D. Selon cette variante de réalisation, les ergots 7 sont de préférence des boutons.

La bande 2 peut être moulée directement sous forme de boucle 20. La bande 2 peut également être moulée sous forme dépliée puis être ultérieurement pliée pour former une boucle 20. De préférence, le ou les éléments de séparation 19 sont moulés ou imprimés avec la bande 2 en une seule pièce, bien qu'il soit possible de mouler séparément la bande et l'élément de séparation, puis de les assembler.

Le polymère utilisé pour le moulage ou l'impression en trois dimensions de la bande 2 ainsi que ses propriétés, notamment ses propriétés mécaniques, sont à choisir et à ajuster en fonction de l'utilisation du dispositif. Par exemple, dans le cadre d'un traitement orthopédique nécessitant une très faible mobilité des doigts blessés voire une immobilisation totale desdits doigts blessés entre eux et/ou par rapport aux autres doigts, on privilégiera un polymère possédant une faible élasticité et une forte rigidité, de préférence le polymère est alors choisi parmi les polymères thermoplastiques ou thermodurcissables. Dans la cadre d'une reprise sportive autorisant une mobilité modérée des doigts blessés entre eux et/ou par rapport aux autres doigts, on privilégiera un polymère possédant au contraire une bonne élasticité et une faible rigidité, de préférence le polymère est alors choisi parmi les élastomères. D'autres paramètres peuvent conditionner le choix du polymère à utiliser, comme par exemple la rigidité dont la valeur influe directement sur la protection des doigts ou des orteils apportée par le dispositif selon l'invention. Enfin, bien qu'il soit préférable d'utiliser le même polymère pour la fabrication de la bande 2 et de l'élément de séparation 19, il est également possible d'utiliser des polymères différents physiquement et/ou chimiquement pour la fabrication de ladite bande et dudit élément de séparation, notamment pour des raisons de confort ou de mobilité des doigts.

## Revendications

1. Dispositif (1) de maintien et/ou de protection d'au moins deux doigts ou orteils permettant d'immobiliser totalement ou partiellement lesdits doigts ou orteils les uns par rapport aux autres, le dispositif comprenant :
une bande (2) se présentant sous la forme d'une boucle (20) délimitant un espace de logement (3) destiné à accueillir et entourer lesdits doigts ou orteils, la bande (2) étant configurée pour épouser la forme des doigts ou orteils lorsque les doigts ou orteils sont insérés dans l'espace de logement (3),
ladite bande (2) se terminant par deux extrémités (5,6) formant deux parties (5,6) d'un système de fermeture (4) de la boucle, les deux parties comportant des pièces d'attaches complémentaires (7,11) coopérant pour assurer un réglage et la fermeture la boucle (20),
dans lequel la bande (2) est en matériau polymère rigide et est munie à l'une de ses extrémités d'ergots (7) et à l'autre extrémité d'orifices (11) constituant les pièces d'attache du système de fermeture.

2. Dispositif selon la revendication 1, dans lequel les ergots (7) sont de type boutons ou crochets sur une première partie (5) du système de fermeture, et
les pièces complémentaires sont disposées de sorte que le système de fermeture (4) permet le réglage de la longueur de la boucle (20) par superposition de la première partie (5) et de la deuxième partie (6) et insertion des ergots (7) dans les orifices (11).

3. Dispositif selon l'une des revendications 1 à 2, dans lequel la première partie (5) du système de fermeture (4) est munie d'une ou plusieurs rangées (10) d'ergots (7), de préférence lesdites rangées d'ergots définissent un axe parallèle à l'axe longitudinal (A) de la bande (2) et dans lequel la deuxième partie (6) du système de fermeture (4) est munie d'une ou plusieurs rangées (12) d'orifices (11), de préférence lesdites rangées d'orifices définissent un axe parallèle à l'axe longitudinal (A) de la bande (2).

4. Dispositif selon l'une des revendications 1 à 2, dans lequel le nombre d'ergots (7) de la première partie (5) du système de fermeture (4) est inférieur ou égal au nombre d'orifices (11) de la deuxième partie (6) du système de fermeture (4).

5. Dispositif selon l'une des revendications 1 à 2, dans lequel les ergots (7) sont des boutons en forme de champignons ou des crochets, et les orifices (11) ont un diamètre au moins égal au diamètre de la tête des champignons ou à la largeur des crochets.

6. Dispositif selon la revendication 1, dans lequel la bande (2) comprend en outre une première partie intermédiaire (14) et une deuxième partie intermédiaire (15), délimitant une partie centrale (13), lesdites parties intermédiaires étant aptes à se courber de manière à constituer les extrémités latérales (17, 18) de la boucle (20).

7. Dispositif selon la revendication 1, dans lequel la bande (2) comprend au moins un élément de séparation (19) des doigts prévu au niveau de la partie centrale (13).

8. Dispositif selon la revendication 7, dans lequel l'élément de séparation (19) des doigts ou orteils comprend deux faces latérales (23) et deux faces longitudinales (22), lesdites faces latérales (23) étant évasées au niveau de leur partie inférieure de manière à former une concavité (25) au niveau de la partie inférieure des deux faces longitudinales (22).

9. Dispositifs selon les revendications 7 et 8, dans lequel l'élément de séparation (19) des doigts ou orteils comprend un retrait (26) réalisé dans la partie inférieure des faces latérales (23) et traversant ledit élément de séparation (19) sur toute sa longueur.

10. Dispositif selon la revendication 1, dans lequel la bande (2) comprend en outre une pluralité d'orifices (16) de faible diamètre comparativement aux orifices (11) du système de fermeture (4), permettant une régulation de la température et de l'humidité au niveau des doigts ou des orteils entourés par ladite bande (2).

11. Dispositif selon la revendication 1, dans lequel la boucle (20) est de forme sensiblement oblongue et de hauteur variable de sorte que la hauteur h de la première extrémité latérale (17) de la boucle (20) est inférieure à la hauteur H de la deuxième extrémité latérale (18) de la boucle (20).

12. Dispositif selon la revendication 1, dans lequel la bande (2) est obtenue par moulage en une seule pièce à partir d'une résine polymère ou par impression en trois dimensions en une seule pièce à partir d'une résine polymère, de sorte que les deux parties (5,6) du système de fermeture font intégralement partie de la bande.

## Patentansprüche

1. Vorrichtung (1) zum Ruhigstellen und/oder Schützen von wenigstens zwei Fingern oder Zehen, so dass die Finger oder Zehen unbeweglich relativ zueinander gehalten werden können, wobei die Vorrichtung Folgendes umfasst:
ein Band (2) in Form einer Schlaufe (20), die einen Aufnahmeraum (3) begrenzt, der zum Aufnehmen und Umgeben der genannten Finger oder Zehen bestimmt ist, wobei das Band (2) so konfiguriert ist, dass es sich der Form der Finger oder Zehen anpasst, wenn die Finger oder Zehen in den Aufnahmeraum (3) gesteckt werden,
wobei das genannte Band (2) an zwei Enden (5,6) endet, die zwei Teile (5,6) eines Systems (4) zum Schließen der Schlaufe bilden, wobei die beiden Teile komplementäre Befestigungsteile (7,11) umfassen, die zusammenwirken, um das Verstellen und Schließen der Schlaufe (20) zu gewährleisten,
wobei das Band (2) aus einem starren Polymermaterial gefertigt und an einem seiner Enden mit Vorsprüngen (7) und am anderen Ende mit Öffnungen (11) versehen ist, die die Befestigungsteile des Schließsystems bilden.

2. Vorrichtung nach Anspruch 1, bei der die Vorsprünge (7) vom Knopf- oder Hakentyp an einem ersten Teil (5) des Schließsystems sind, und
die komplementären Teile so angeordnet sind, dass das Schließsystem (4) das Verstellen der Länge der Schlaufe (20) durch Übereinanderlegen des ersten Teils (5) und des zweiten Teils (6) und Einstecken der Vorsprünge (7) in die Öffnungen (11) zulässt.

3. Vorrichtung nach einem der Ansprüche 1 bis 2, bei der der erste Teil (5) des Schließsystems (4) mit einer oder mehreren Reihen (10) von Vorsprüngen (7) versehen ist, wobei die genannten Reihen von Vorsprüngen vorzugsweise eine Achse parallel zur Längsachse (A) des Bandes (2) definieren, und bei der der zweite Teil (6) des Schließsystems (4) mit einer oder mehreren Reihen (12) von Öffnungen (11) versehen ist, und wobei die genannten Öffnungsreihen vorzugsweise eine Achse parallel zur Längsachse (A) des Bandes (2) definieren.

4. Vorrichtung nach einem der Ansprüche 1 bis 2, bei der die Anzahl von Vorsprüngen (7) des ersten Teils (5) des Schließsystems (4) gleich oder kleiner ist als die Anzahl von Öffnungen (11) des zweiten Teils (6) des Schließsystems (4).

5. Vorrichtung nach einem der Ansprüche 1 bis 2, bei der die Vorsprünge (7) Knöpfe in Form von Pilzen oder Haken sind und die Öffnungen (11) einen Durchmesser wenigstens gleich dem Durchmesser des Kopfs der Pilze oder der Breite der Haken haben.

6. Vorrichtung nach Anspruch 1, bei der das Band (2) ferner einen ersten Zwischenteil (14) und einen zweiten Zwischenteil (15) umfasst, die einen mittleren Teil (13) begrenzen, wobei sich die Zwischenteile auf eine solche Weise krümmen können, dass sie die lateralen Enden (17,18) der Schlaufe (20) bilden.

7. Vorrichtung nach Anspruch 1, bei der das Band (2) wenigstens ein Element (19) zum Trennen der Finger umfasst, das an dem mittleren Teil (13) vorgesehen ist.

8. Vorrichtung nach Anspruch 7, wobei das Element (19) zum Trennen der Finger oder Zehen zwei laterale Flächen (23) und zwei longitudinale Flächen (22) aufweist, wobei die genannten lateralen Flächen (23) an ihrem unteren Teil so aufgeweitet sind, dass sie eine Konkavität (25) am unteren Teil der beiden longitudinalen Flächen (22) bilden.

9. Vorrichtung nach den Ansprüchen 7 und 8, bei der das Element (19) zum Trennen der Finger oder Zehen eine Aussparung (26) aufweist, die im unteren Teil der lateralen Flächen (23) realisiert ist und das Trennelement (19) über seine gesamte Länge überquert.

10. Vorrichtung nach Anspruch 1, bei der das Band (2) ferner mehrere Öffnungen (16) mit einem kleinen Durchmesser im Vergleich zu den Öffnungen (11) des Schließsystems (4) umfasst, die eine Regelung der Temperatur und Feuchtigkeit an den Fingern oder Zehen zulässt, die von dem genannten Band (2) umgeben sind.

11. Vorrichtung nach Anspruch 1, bei der die Schlaufe (20) eine im Wesentlichen längliche Form und eine variable Höhe aufweist, so dass die Höhe h des ersten lateralen Endes (17) der Schlaufe (20) kleiner ist als die Höhe H des zweiten lateralen Endes (18) der Schlaufe (20).

12. Vorrichtung nach Anspruch 1, bei der das Band (2) durch einstückiges Formen aus einem Polymerharz oder durch dreidimensionales Drucken aus einem Stück aus einem Polymerharz erhalten wird, so dass die beiden Teile (5,6) des Schließsystems einen integralen Bestandteil des Bandes bilden.

## Claims

1. Device (1) for holding in position and/or protecting at least two fingers or toes, enabling said fingers or toes to be totally or partly immobilised relative to one another, the device comprising:
a strip (2) which is in the form of a loop (20) which defines an accommodation space (3) intended to receive and surround said fingers or toes, the strip (2) being configured to follow the shape of the fingers or toes when said fingers or toes are inserted in the accommodation space (3),
said strip (2) being terminated by two ends (5,6) which form two portions (5,6) of a closure system (4) for the loop, the two portions comprising complementary fastening parts (7,11) which co-operate to enable the loop (20) to be adjusted and closed,
wherein the strip (2) is made of rigid polymer material and is provided at one of its ends with projections (7) and at the other end with openings (11), which form the fastening parts of the closure system.

2. Device according to claim 1, wherein the projections (7) are of the button or hook type on a first portion (5) of the closure system, and
the complementary parts are so arranged that the closure system (4) enables the length of the loop (20) to be adjusted by laying the first portion (5) and the second portion (6) one on top of the other and inserting the projections (7) in the openings (11).

3. Device according to either of claims 1 to 2, wherein the first portion (5) of the closure system (4) is provided with one or more rows (10) of projections (7), and said rows of projections preferably define an axis parallel to the longitudinal axis (A) of the strip (2), and wherein the second portion (6) of the closure system (4) is provided with one or more rows (12) of openings (11), and said rows of openings preferably define an axis parallel to the longitudinal axis (A) of the strip (2).

4. Device according to either of claims 1 to 2, wherein the number of projections (7) on the first portion (5) of the closure system (4) is equal to or less than the number of openings (11) in the second portion (6) of the closure system (4).

5. Device according to either of claims 1 to 2, wherein the projections (7) are buttons in the shape of mushrooms, or hooks, and the openings (11) are of a diameter at least equal to the diameter of the heads of the mushrooms or to the width of the hooks.

6. Device according to claim 1, wherein the strip (2) also comprises a first intermediate portion (14) and a second intermediate portion (15), which define a central portion (13), said intermediate portions being able to be curved in such a way as to form the lateral ends (17,18) of the loop (20) .

7. Device according to claim 1, wherein the strip (2) comprises at least one member (19) for separating the fingers which is provided at the central portion (13).

8. Device according to claim 7, wherein the member (19) for separating the fingers or toes comprises two end faces (23) and two longitudinal faces (22), said end faces being (23) flared in the region of their lower portions in such a way as to create a concavity (25) in the region of the lower portions of the two longitudinal faces (22).

9. Device according to claims 7 and 8, wherein the member (19) for separating the fingers or toes comprises a recess (26) which is made in the bottom portion of the lateral faces (23) and which passes through said separating member (19) for the whole of its length.

10. Device according to claim 1, wherein the strip (2) also comprises a plurality of openings (16) of small diameter in comparison with the openings (11) in the closure system (4), which enable temperature and humidity to be regulated in the region of the fingers or toes surrounded by said strip (2) .

11. Device according to claim 1, wherein the loop (20) is substantially oblong in shape and variable in height such that the height h of the first lateral end (17) of the loop (20) is less than the height H of the second lateral end (18) of the loop (20).

12. Device according to claim 1, wherein the strip (2) is obtained by one-piece moulding from a polymer resin or by one-piece 3D printing from a polymer resin, such that the portions (5,6) of the closure system form an integral part of the strip.
